# EUROPÄISCHE PATENTSCHRIFT

(11) **EP 2 322 142 B1**
(45) Veröffentlichungstag und Bekanntmachung des Hinweises auf die Patenterteilung: **27.07.2016**
(21) Anmeldenummer: 09175794.8
(22) Anmeldetag: 12.11.2009
(51) Int. Cl.: A61K 9/00, A61K 9/51

(54) **Biokompatible, magnetische Nanopartikel zur Behandlung von Glioblastomen**
Bio-compatible, magnetic nano-particles for handling glioblastoma
Nanoparticules biocompatibles magnétiques pour le traitement de glioblastomes

(43) Veröffentlichungstag der Anmeldung: 18.05.2011
(73) Patentinhaber: Helmholtz-Zentrum Geesthacht Zentrum für Material- und Küstenforschung GmbH, 21502 Geesthacht (DE)
(72) Erfinder: Mucha, Birte, 21493 Schwarzenbek (DE); Willumeit, Regine, 22041 Hamburg (DE); Lamszus, Katrin, 22455 Hamburg (DE)
(74) Vertreter: Uexküll & Stolberg

(56) Entgegenhaltungen:
- WO-A1-2009/070282
- WO-A2-2006/108405
- WO-A2-2009/156743
- DE-A1-102008 008 522

## Beschreibung

Die vorliegende Erfindung betrifft die Verwendung biokompatibler, magnetischer Nanopartikel bei der Therapie von Glioblastomen.

### Stand der Technik

Das Glioblastom (*Glioblastoma multiforme*) ist der am häufigsten auftretende bösartige Hirntumor bei Erwachsenen. Ungefähr 15% bis 30% aller hirneigenen Tumore sind Glioblastome. Das Glioblastom weist feingewebliche Ähnlichkeiten mit den Gliazellen des Gehirns auf und wird aufgrund der sehr schlechten Prognose nach der WHO-Klassifikation der Tumoren des zentralen Nervensystems als Grad IV eingestuft. Die Behandlung besteht in operativer Reduktion der Tumormasse, Bestrahlung und Chemotherapie. Eine endgültige Heilung kann aber derzeit nicht erreicht werden. Die mittlere Überlebenszeit liegt in der Größenordnung von sechs Monaten.

Glioblastome können völlig neu (*de novo*) oder durch fortschreitende Entdifferenzierung aus weniger bösartigen Astrozytomen entstehen. Daher kommt es nicht selten vor, dass therapierte Astrozytome sich im Rezidiv als Glioblastom manifestieren. Diese sogenannten sekundären Glioblastome treten eher bei jüngeren Patienten auf.

Kennzeichnend für Glioblastome ist ein diffuses, infiltratives und sehr schnelles Wachstum. Eine kurzfristige klinische Besserung kann durch Behandlung des praktisch immer vorhandenen Hirnödems mit Dexamethason erreicht werden. Die neurochirurgische Operation mit Verminderung (Reduktion) der Hauptmasse des Tumors kann das Fortschreiten der Erkrankung verlangsamen, aber nicht dauerhaft verhindern, da praktisch immer einzelne Tumorzellen das gesunde Gehirngewebe schon infiltrativ durchwandert haben und deswegen eine vollständige Tumorentfernung nicht möglich ist. Zur Verlängerung der rezidivfreien und absoluten Überlebenszeit schließt sich deswegen an die Operation praktisch immer eine Bestrahlung und häufig auch eine Chemotherapie an.

Neben den bereits genannten Behandlungen, werden in jüngerer Zeit ebenfalls magnetische Nanopartikel zur Therapie von Glioblastomen eingesetzt. In diesem Zusammenhang wurden magnetische Nanopartikel beispielsweise zur zielgerichteten Einschleusung von Wirksubstanzen in Zellen (drug targeting) oder zur Zerstörung krankhafter Zellen im magnetischen Wechselfeld (Hyperthermie) verwendet.

Ein Verfahren zur Einschleusung von Wirksubstanzen in Zellen mit Hilfe von multifunktionellen Nanopartikeln unter Einfluss eines externen magnetischen Wechselfeldes und zur Zerstörung der Zellen durch Hyperthermie wurde in WO 2005/065282 A2 beschrieben. Es wurden zwei Verfahren zur Zerstörung der krankhaften Zellen beschrieben, die beide mit Hilfe eines magnetischen Wechselfeldes ablaufen, nämlich (1) magnetomechanische Zellzerstörung und (2) magnetisch aktivierte Wirkstofffreisetzung.

Die Verwendung von magnetischen Nanopartikeln als Kontrastmittel zur Diagnose wurde in DE 4428851 A1 beschrieben. Dazu werden Eisen enthaltende, magnetische Teilchen verwendet, die gegebenenfalls pharmazeutische Hilfsstoffe und/oder Adsorptionsvermittler enthalten verwendet. Dabei wird ein magnetisches Wechselfeld zur Ausrichtung und Umorientierung der magnetischen Dipole der Teilchen angelegt.

Nanopartikel-enthaltende implantierbare Produkte und deren Verwendung, insbesondere zur thermotherapeutischen Nachbehandlung nach chirurgischer Entfernung von Tumoren und Krebsgeschwüren wurden in DE 10 2008 008 522 A1 beschrieben. Die verwendeten Nanopartikel haben bevorzugt einen magnetischen, besonders bevorzugt einen superparamagnetischen Kern, sodass sie in einem magnetischen Wechselfeld erwärmt werden und somit zur Hyperthermie verwendet werden können. Bevorzugte Materialien sind zum Beispiel Maghämit, Magnetit, Eisen-Nickel-Legierungen oder Nickel-Kupfer-Legierungen.

Die Verwendung von magnetischen Mikroblasen zur gezielten Abgabe von therapeutischen Wirkstoffen wurde in WO 2009/156743 beschrieben. Die magnetischen Mikroblasen bestehen aus einem Gaskern und einer flüssigen Hülle, die magnetische Nanopartikel, bevorzugt ferromagnetische Nanopartikel wie Eisenoxid, enthält. Durch Anlegen eines magnetischen Wechselfeldes können die magnetischen Mikroblasen zielgerichtet in Zellen eingeschleust werden.

Aufgabe der vorliegenden Erfindung ist es neuartige Stoffe für die zielgerichtete Bewegung von Glioblastomen zur Verfügung zu stellen. Die Bewegung der Zellen soll erstmalig im Magnetfeld möglich werden.

### Beschreibung der Erfindung

Die Aufgabe wird durch biokompatible, magnetische Nanopartikel für die Bewegung von Glioblastomen in einem statischen Magnetfeld gelöst. Unter magnetischen Nanopartikeln versteht man magnetisierbare Teilchen, deren hydrodynamische Größe weniger als 1 µm, meist weniger als 500 nm beträgt und vorzugsweise im Bereich im Bereich von 5 nm bis 300 nm, besonders bevorzugt im Bereich von 50 nm bis 200 nm liegt. Der Kerndurchmesser beträgt vorzugsweise 1 nm bis 300 nm, bevorzugter 2 nm bis 100 nm und insbesondere 3 nm bis 50 nm. Die Größe der magnetischen Nanopartikel liegt somit im Bereich der Größe eines Proteins (5 bis 50 nm) oder eines Virus (20 bis 450 nm).

Als magnetisierbare Teilchen kommen in erster Linie Metalle sowie Oxide der achten Nebengruppe des Periodensystems der Elemente in Frage. Bevorzugt ist das Material, aus denen die biokompatiblen, magnetischen Nanopartikel bestehen, aus Eisen (Fe), Gadolinium (Gd) oder deren Oxiden ausgewählt. Insbesondere bevorzugte Materialien sind Magnetit oder seine oxidierte Form, das Maghemit.

Der Kern der magnetischen Nanopartikel ist vorzugsweise von einer Hülle umgeben, wobei oberflächenaktive Substanzen an deren Oberfläche adsorbiert oder chemisorbiert sind. Diese Hülle soll verhindern, dass die Partikel agglomerieren oder sedimentieren können. Durch die Hüllschicht erhöht sich der Gesamtdurchmesser der Partikel, welcher zusätzlich noch durch die Wasserbindungskapazität der Hüllmaterialien vergrößert wird. Daher wird der Gesamtdurchmesser in wässriger Lösung als hydrodynamischer Durchmesser angegeben.

Als Hüllmaterialien der Nanopartikel können verschiedene Substanzen verwendet werden. Für eine Anwendung im medizinischen Bereich müssen diese aber für den Menschen bioverträglich sein. Als Hüllmaterialien werden vorzugsweise Polymere wie Dextran, Carboxydextran, Polyethylenglycol, Stärke oder Albumin verwendet. Werden nicht Polymere, sondern biomimetische Monomere wie Lipide, Fettsäuren, Citrat, Myristinsäure oder Laurinsäure als Hülle gewählt, so können noch kleinere Partikel hergestellt werden.

Besonders bevorzugt sind natürlich vorkommende, biokompatible, magnetische Nanopartikel wie Magnetosomen aus magnetotaktischen Bakterien, die die Fähigkeit zur Synthese von intrazellulären, membranumschlossenen Partikeln aus Magnetit besitzen. Insbesondere werden Magnetosomen aus *Magnetospirillum gryphiswaldense* MSR-1, *Magnetospirillium magnetotacticum, Magnetospirillium* spec. AMB-1, magnetischem Kokkus MC-1 oder magnetischem Vibrio MC-1 verwendet. Magnetotaktische Bakterien sind dem Fachmann bekannt und werden beispielsweise in Schüler, D., and Köhler, M. (1992) "The isolation of a new magnetic spirillum" Zentralbl. Mikrobiol. 147: 150-151, Bazylinski, D.A., Frankel, R.B., und Jannasch, H.W. (1988) "Anaerobic magnetite production by a marine, magnetotactic bacterium" Nature 334: 518-519, Kawaguchi, R., Burgess, J.G., und Matsunaga, T. (1992) "Phylogeny and 16s rRNA sequence of Magnetospirillum sp. AMB-1, an aerobic magnetic bacterium" Nucleic. Acids. Res. 20: 1140, Meldrum, F.C., Mann, S., Heywood, B.R., Frankel, R.B., und Bazylinski, D.A. (1993) "Electron-microscopy study of magnetosomes in a cultured coccoid magnetotactic bacterium" P. Roy. Soc. Lond. B. Bio. 251: 231-236, Meldrum, F.C., Mann, S., Heywood, B.R., Frankel, R.B., und Bazylinski, D.A. (1993) "Electron-microscopy study of magnetosomes in 2 cultured vibrioid magnetotactic bacteria" P. Roy. Soc. Lond. B. Bio. 251: 237-242 sowie Schleifer, K., Schüler, D., Spring, S., Weizenegger, M., Amann, R., Ludwig, W. und Köhler, M. (1991) "The genus Magnetospirillum gen. nov., description of Magnetospirillum gryphiswaldense sp. nov. and transfer of Aquaspirillum magnetotacticum to Magnetospirillum magnetotacticum comb. nov." Syst. Appl. Microbiol. 14: 379-385 beschrieben, auf die hier Bezug genommen wird.

In der Diagnostik pathologischer Prozesse werden die erfindungsgemäßen magnetischen Nanopartikel bereits seit einigen Jahren eingesetzt. Von großer Bedeutung ist ihr Einsatz als Kontrastmittel in der Magnetresonanz-Tomographie (MRT). Bislang zugelassene MRT-Kontrastmittel für Leber und Milz sind unter den Handelsnamen Endorem^{®}) oder Resovist^{®}) erhältlich, mit denen sich Lebermetastasen und gering differenzierte Lebertumore, denen Makrophagen fehlen, gut darstellen lassen. Resovist^{®} ist ein Ferrofluid mit einen hydrodynamischen Durchmesser von ca. 60 nm und einen Kerndurchmesser von 3 nm bis 15 nm. Mittlerweile sind neuere Ferrofluide erhältlich, welche aus größeren Partikeln mit hydrodynamischen Durchmessern von 120 nm bis 150 nm bestehen.

Besonders bevorzugt werden die erfindungsgemäßen biokompatiblen, magnetischen Nanopartikel von den Zellen aufgenommen.

Erfindungsgemäß werden die biokompatiblen, magnetischen Nanopartikel zur zielgerichteten Bewegung der migrierenden Krebszellen in einem externen Magnetfeld (*Magnetotaxis*) verwendet, um sie gesammelt einem chirurgischen Eingriff oder Hyperthermie zugänglich zu machen.

Eine mögliche Applikationsform von magnetischen Nanopartikeln besteht in der Gabe direkt in das Gehirn, um zu vermeiden, dass die Nanopartikel durch die Blut-Hirn-Schranke aufgehalten werden. Die mit Partikeln beladenen Zellen können nach der Gabe dann durch Anlegen eines äußeren Magnetfeldes an die Zielregion dirigiert werden.

Eine weitere, bevorzugte Möglichkeit besteht darin, spezifische Antikörper an die Nanopartikel zu koppeln, die an Antigene in der befallenen Region binden. Die Antikörper binden vorzugsweise spezifisch an Oberflächenantigene von Glioblastom-Zellen, ohne dass gesundes Gewebe betroffen wird.

## Patentansprüche

1. Biokompatible, magnetische Nanopartikel zur Anwendung bei der Therapie von Glioblastomen im statischen Magnetfeld, wobei die biokompatiblen, magnetischen Nanopartikel zur zielgerichteten Bewegung der migrierenden Krebszellen in einem externen Magnetfeld (Magnetotaxis) verwendet werden, um sie gesammelt einem chirurgischen Eingriff oder Hyperthermie zugänglich zu machen.

2. Nanopartikel nach Anspruch 1, **dadurch gekennzeichnet, dass** sie eine hydrodynamische Größe von weniger als 1 µm, vorzugsweise im Bereich im Bereich von 5 nm bis 300 nm, besonders bevorzugt im Bereich von 50 nm bis 200 nm aufweisen.

3. Nanopartikel nach einem der vorgehenden Ansprüche, **dadurch gekennzeichnet, dass** sie einen Kerndurchmesser von 1 nm bis 300 nm, vorzugsweise 3 nm bis 50 nm aufweisen.

4. Nanopartikel nach einem der vorgehenden Ansprüche, **dadurch gekennzeichnet, dass** sie aus Eisen (Fe), Gadolinium (Gd) oder deren Oxiden ausgewählt sind.

5. Nanopartikel nach Anspruch 4, **dadurch gekennzeichnet, dass** sie aus Magnetit oder Maghemit ausgewählt sind.

6. Nanopartikel nach einem der vorgehenden Ansprüche, **dadurch gekennzeichnet, dass** sie ein Hüllmaterial aufweisen.

7. Nanopartikel nach Anspruch 6, **dadurch gekennzeichnet, dass** das Hüllmaterial aus Polymeren wie Dextran, Carboxydextran, Polyethylenglycol, Stärke, Albumin oder einem biomimetischem Material wie Lipiden, Fettsäuren wie Myristinsäure oder Laurinsäure, oder Citrat, ausgewählt ist.

8. Nanopartikel nach einem der Ansprüche 6 oder 7, **dadurch gekennzeichnet, dass** das Hüllmaterial zusätzlich Antikörper enthält.

9. Nanopartikel nach Anspruch 8, **dadurch gekennzeichnet, dass** der oder die Antikörper an Oberflächenantigene von Glioblastom-Zellen spezifisch bindet.

10. Nanopartikel nach einem der vorgehenden Ansprüche, **dadurch gekennzeichnet, dass** sie Magnetosomen aus magnetostatischen Bakterien sind.

11. Nanopartikel nach Anspruch 6, **dadurch gekennzeichnet, dass** die Magnetosomen aus *Magnetospirillum gryphiswaldense* MSR-*1, Magnetospirillium magnetotacticum, Magnetospirillium* spec. AMB-1, magnetischem Kokkus MC-1 oder magnetischem Vibrio MC-1 erhältlich sind.

## Claims

1. Biocompatible, magnetic nanoparticles for use in the therapy of glioblastomae in a static magnetic field, wherein the biocompatible magnetic nanoparticles are used for a targeted movement of migrating cancer cells in an external magnetic field (magnetotaxis) for collectively making them accessible for surgical intervention or hyperthermia.

2. Nanoparticles according to claim 1, **characterized in that** said nanoparticles have a hydrodynamic size of less than 1 µm, preferably in the range from 5 nm to 300 nm, particularly preferably in the range from 50 nm to 200 nm.

3. Nanoparticles according to any of the previous claims, **characterized in that** said nanoparticles have a core diameter of 1 nm to 300 nm, preferably of 3 nm to 50 nm.

4. Nanoparticles according to any of the previous claims, **characterized in that** said nanoparticles are selected from iron (Fe), gadolinium (Gd) or the oxides thereof.

5. Nanoparticles according to claim 4, **characterized in that** said nanoparticles are selected from magnetite or maghemite.

6. Nanoparticles according to any of the previous claims, **characterized in that** said nanoparticles comprise a shell material.

7. Nanoparticles according to claim 6, **characterized in that** the shell material is selected from polymers such as dextran, carboxydextran, polyethylene glycol, starch, albumin, or a biomimetic material such as lipids, fatty acids such as myristic acid or lauric acid, or citrate.

8. Nanoparticles according to any of claims 6 or 7, **characterized in that** the shell material additionally comprises antibodies.

9. Nanoparticles according to claim 8, **characterized in that** the antibody or antibodies specifically binds/bind to surface antigens of glioblastoma cells.

10. Nanoparticles according to any of the previous claims, **characterized in that** said nanoparticles are magnetosomes from magnetostatic bacteria.

11. Nanoparticles according to claim 6, **characterized in that** the magnetosomes are obtainable from *Magnetospirillum gryphiswaldense* MSR-1, *Magnetospirillium magnetotacticum, Magnetospirillium* spec. AMB-1, magnetic coccus MC-1 or magnetic *Vibrio* MC-1.

## Revendications

1. Nanoparticules magnétiques biocompatibles pour utilisation dans la thérapie des glioblastomes dans un champ magnétique statique, lesquelles nanoparticules magnétiques biocompatibles sont utilisées pour induire un déplacement ciblé de cellules cancéreuses migratrices dans un champ magnétique externe (magnétotaxie) en vue de les rendre collectivement accessibles pour une intervention chirurgicale ou pour une hyperthermie.

2. Nanoparticules conformes à la revendication 1, **caractérisées en ce qu'**elles présentent une taille hydrodynamique inférieure à 1 µm, située de préférence dans l'intervalle allant de 5 nm à 300 nm, et mieux encore dans l'intervalle allant de 50 nm à 200 nm.

3. Nanoparticules conforme à l'une des revendications précédentes, **caractérisées en ce qu'**elles présentent un diamètre de coeur de 1 nm à 300 nm, et de préférence de 3 nm à 50 nm.

4. Nanoparticules conforme à l'une des revendications précédentes, **caractérisées en ce qu'**elles sont choisies parmi du fer (Fe), du gadolinium (Gd) et les oxydes de ces métaux.

5. Nanoparticules conformes à la revendication 4, **caractérisées en ce qu'**elles sont choisies parmi de la magnétite et de la maghémite.

6. Nanoparticules conforme à l'une des revendications précédentes, **caractérisées en ce qu'**elles comportent un matériau de coque.

7. Nanoparticules conformes à la revendication 6, **caractérisées en ce que** le matériau de coque est choisi parmi des polymères comme les dextrane, carbodextrane, polyéthylèneglycol, amidon, albumine, ou est un matériau biomimétique, comme les lipides, les acides gras, tel l'acide myrisytique ou l'acide laurique, ou le citrate.

8. Nanoparticules conformes à la revendication 6 ou 7, **caractérisées en ce que** le matériau de coque comporte en outre des anticorps.

9. Nanoparticules conformes à la revendication 8, **caractérisées en ce que** l'anticorps ou les anticorps se lie(nt) spécifiquement aux antigènes de surface des cellules de glioblastome.

10. Nanoparticules conforme à l'une des revendications précédentes, **caractérisées en ce qu'**elles sont des magnétosomes issus de bactéries magnétostatiques.

11. Nanoparticules conformes à la revendication 6, **caractérisées en ce que** les magnétosomes peuvent être obtenus à partir de *Magnetospirillum gryphiswaldense* MSR-1, *Magrtetospirillum magnetotacticum*, *Magnetospirillum* spec. AMB-1, *Coccus* magnétique MC-1 ou *Vibrio* magnétique MC-1.
